# EUROPÄISCHE PATENTSCHRIFT

(11) **EP 1 250 307 B1**
(45) Veröffentlichungstag und Bekanntmachung des Hinweises auf die Patenterteilung: **21.05.2008**
(21) Anmeldenummer: 01946840.4
(22) Anmeldetag: 24.01.2001
(51) Int. Cl.: C07C 51/09, C07C 51/48, C07C 53/02

(54) **VERWENDUNG EINES EXTRAKTIONSMITTELS ALS ANTISCHAUMMITTEL BEI DER HERSTELLUNG VON WASSERFREIER AMEISENSÄURE**
UTILIZATION OF AN EXTRACTING AGENT AS ANTIFOAMING AGENT IN THE PRODUCTION OF ANHYDROUS FORMIC ACID
UTILISATION D'UN AGENT D'EXTRACTION EN TANT QU'AGENT ANTI-MOUSSE LORS DE LA PRODUCTION D'ACIDE FORMIQUE ANHYDRE

(30) Priorität: 24.01.2000 DE 10002793
(43) Veröffentlichungstag der Anmeldung: 23.10.2002
(73) Patentinhaber: BASF SE, 67056 Ludwigshafen (DE)
(72) Erfinder: AUER, Heinz, 68809 Neulussheim (DE); BESSLING, Bernd, Grosse Ille, MI 48138 (US); HAMMER, Hans, 68219 Mannheim (DE); HASSE, Hans, 67661 Kaiserslautern (DE); SAUER, Friedrich, 67271 Obersülzen (DE); VICARI, Maximilian, 67117 Limburgerhof (DE); WAGNER, Gerhard, 67069 Ludwigshafen (DE); ADRIAN, Till, 67240 Bobenheim-Roxheim (DE)
(74) Vertreter: Isenbruck, Günter
(86) Internationale Anmeldenummer: PCT/EP2001/000749
(87) Internationale Veröffentlichungsnummer: WO 2001/055071

(56) Entgegenhaltungen:
- EP-A- 0 017 866
- EP-A- 0 156 309

## Beschreibung

Die vorliegende Erfindung betrifft eine Vorrichtung und ein Verfahren zur Herstellung von wasserfreier oder weitgehend wasserfreier Ameisensäure sowie die Verwendung des bei der Aufarbeitung der Ameisensäure eingesetzten Extraktionsmittels.

Aus "Ullmanns Encyklopädie der technischen Chemie", 4. Auflage, Band 7, Seite 365, ist bekannt, Ameisensäure durch Acydolyse von Formamid mit Schwefelsäure herzustellen. Dieses Verfahren hat jedoch den Nachteil, daß stöchiometrische Mengen Ammoniumsulfat als Zwangsanfall erhalten werden.

Ein weitere Möglichkeit zur Herstellung von Ameisensäure besteht in der Hydrolyse von Methylformiat, das aus Methanol und Kohlenmonoxid synthetisiert wird. Dabei liegen die folgenden Gleichungen zugrunde:

| | | | |
|---|---|---|---|
| | CO + CH₃-OH | → | CH₃-O-CO-H |
| | CH₃-O-CO-H+H₂O | → | CH₃-OH+H-CO-OH |
| Brutto: | CO+H₂O | → | H-CO-OH |

Die in "Ullmanns Encyklopädie der technischen Chemie", 4. Auflage, Band 7, Seite 366 beschriebene Hydrolyse von Methylformiat

HCOOCH₃ + H₂O HCOOH + CH₃OH

hat den Nachteil einer ungünstigen Lage des Hydrolysegleichgewichtes. Eine Gleichgewichtsverschiebung durch destillative Entfernung der gewünschten Verfahrensprodukte ist nicht möglich, da Methylformiat (Sdp. 32°C) wesentlich niedriger als Methanol (Sdp. 65°C) und Ameisensäure (Sdp. 101°C) siedet. Aus der anfallenden wäßrigen Ameisensäurelösung kann wasserfreie Ameisensäure wegen Azeotropbildung mit Wasser nicht ohne weiteres destillativ gewonnen werden. Die Schwierigkeit besteht also darin, aus dem Hydrolysegemisch des Methylformiats wasserfreie Ameisensäure zu gewinnen.

Ein in der EP-B-0 017 866 beschriebenes, die Verfahrensschritte a) bis g) aufweisendes Verfahren ermöglicht ausgehend von Methylformiat die Herstellung wasserfreier Ameisensäure. Dabei erhält man wasserfreie Ameisensäure, falls man
a) Methylformiat der Hydrolyse unterwirft,
b) vom erhaltenen Hydrolysegemisch Methanol sowie überschüssiges Methylformiat abdestilliert,
c) das Ameisensäure und Wasser enthaltende Sumpfprodukt der Destillation (b) in einer Flüssig-flüssig-Extraktion mit einem hauptsächlich die Ameisensäure aufnehmenden Extraktionsmittel extrahiert,
d) die hierbei erhaltene Ameisensäure, Extraktionsmittel und eine Teilmenge des Wassers aufweisende Extraktphase einer Destillation unterwirft,
e) das bei dieser Destillation erhaltene Wasser und eine Teilmenge der Ameisensäure enthaltende Kopfprodukt in den unteren Teil der Destillationskolonne der Stufe (b) zurückführt,
f) das vorwiegend Extraktionsmittel und Ameisensäure enthaltende Sumpfprodukt der Destillationsstufe (d) destillativ in wasserfreie Ameisensäure und das Extraktionsmittel auftrennt und
g) das die Stufe (f) verlassende Extraktionsmittel in den Verfahrensgang zurückführt.

Es ist bei diesem Verfahren besonders zweckmäßig,
h) die Destillationsschritte (b) und (d) in einer einzigen Kolonne vorzunehmen,
i) das für die Hydrolyse benötigte Wasser dampfförmig in den unteren Teil der für die Durchführung von Schritt (b) vorgesehenen Kolonne einzubringen,
k) Methylformiat und Wasser bei der Hydrolyse (a) im Molverhältnis 1:2 bis 1:10 einzusetzen und/oder
l) als Extraktionsmittel ein Carbonsäureamid der allgemeinen Formel I 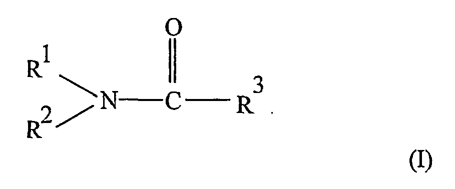 einzusetzen, in der die Reste R¹ und R² Alkyl-, Cycloalkyl-, Aryl- oder Aralkylgruppen bedeuten oder R¹ und R² gemeinsam zusammen mit dem N-Atom einen heterocyclischen 5- oder 6-Ring ausbilden und daß nur einer der Reste eine Arylgruppe ist, in der R³ für Wasserstoff oder eine C₁-C₄-Alkylgruppe steht.

Im folgenden werden die Verfahrensschritte (a) bis (i) des vorstehend beschriebenen, aus der EP-B-0 017 866 bekannten Verfahrens näher erläutert.

### Verfahrensschritt (a)

Die Hydrolyse wird üblicherweise in einem Temperaturbereich von 80 bis 150°C durchgeführt.

### Verfahrensschritt (b)

Die Destillation des Hydrolysegemisches kann prinzipiell bei beliebigem Druck, bevorzugt 0,5 bis 2 bar, vorgenommen werden. Im allgemeinen empfiehlt sich das Arbeiten unter Normaldruck. In diesem Fall beträgt die Temperatur im Kolonnensumpf etwa 110°C und am Kolonnenkopf etwa 30 bis 40°C. Das Hydrolysegemisch wird zweckmäßigerweise in einem Temperaturbereich von 80 bis 150°C zugegebenen, und das Methanol entnimmt man vorzugsweise flüssig bei Temperaturen von 55 bis 65°C. Eine zufriedenstellende Trennung des Gemisches in Methylformiat sowie Methanol einerseits und die wäßrige Ameisenäure andererseits ist bereits mit einer Destillationskolonne möglich, die 25 theoretischen Böden aufweist (bevorzugt ist eine theoretische Bodenzahl von 35 bis 45). Die Bauart der für den Verfahrensschritt (b) vorgesehenen Kolonne kann beliebig sein, besonders empfiehlt sich jedoch eine Siebboden- oder Füllkörper-Kolonne.

### Verfahrensschritt (c)

Die Flüssig-flüssig-Extraktion der Ameisensäure aus ihrer wäßrigen Lösung mittels eines Extraktionsmittels wird vorzugsweise bei Normaldruck und bei Temperaturen von 60 bis 120, insbesondere 70 bis 90°C im Gegenstrom vorgenommen. Je nach Art des Extraktionsmittels benötigt man in der Regel Extraktionseinrichtungen mit 1 bis 12 theoretischen Trennstufen. Geeignete Extraktionseinrichtungen dafür sind insbesondere Flüssig-flüssig-Extraktionskolonnen. In den meisten Fällen erzielt man mit 4 bis 6 theoretischen Trennstufen befriedigende Ergebnisse.

Die Wahl des Extraktionsmittels ist nicht beschränkt. Als Extraktionsmittel besonders geeignet sind Carbonsäureamide der vorstehend genannten allgemeinen Formel I. Derartige Extraktionsmittel sind vor allem N-Di-n-butylformamid sowie außerdem N-Din-butylacetamid, N-Methyl-N-2-heptylformamid, N-n-Butyl-N-2-ethylhexylformamid, N-n-Butyl-N-cyclohexylformamid und N-Ethylformanilid sowie Gemische dieser Verbindungen. Weitere geeignete Extraktionsmittel sind u.a. Diisopropylether, Methylisobutylketon, Ethylacetat, Tributylphosphat und Butandiolformiat.

### Verfahrensschritt (d)

Die Extraktphase wird in einer entsprechenden Destillationseinrichtung destillativ in eine Flüssigphase, die in der Regel vorwiegend Ameisensäure und Extraktionsmittel aufweist, sowie eine vorwiegend Wasser und geringe Mengen Ameisensäure aufweisende Dampfphase getrennt. Es handelt sich dabei um eine Extraktivdestillation. Die Sumpftemperatur beträgt vorzugsweise 140 bis 180°C. Ein ausreichender Trenneffekt wird in der Regel ab 5 theoretischen Böden erzielt.

### Verfahrensschritt (e)

Die Rückführung des Ameisensäure-Wassergemischs erfolgt in der Regel dampfförmig.

### Verfahrensschritte (f) und (g)

Die Destillationseinrichtung (meist als Kolonne ausgebildet) zur Durchführung der Stufe (f) wird zweckmäßigerweise unter vermindertem Druck - etwa 50 bis 300 mbar und entsprechend niedrigen Kopftemperaturen - etwa 30 bis 60°C, betrieben.

### Verfahrensschritt (h)

Diese Variante des Verfahrens betrifft die Schritte (b) und (d). Die Destillationseinrichtungen zur Durchführung der Stufen b) und d) werden in einer Gesamtdestillationseinrichtung angeordenet. Die Destillationseinrichtungen sind dabei in der Regel als Kolonnen ausgebildet.

### Verfahrensschritt (i)

In diesem Schritt wird für die Hydrolyse benötigtes Wasser in Form von Wasserdampf bereitgestellt.

In dem vorstehend beschriebenen Verfahren wird Methylformiat in einem Hydrolysereaktor bei molarem Wasserüberschuß hydrolysiert. Die Hydrolysereaktion findet bevorzugt als reine Flüssigphasenreaktion bei Temperaturen zwischen 80 bis 150°C statt. Um diese Temperaturen der Reaktionsmischung erreichen zu können, muß die Hydrolyse bei Überdruck durchgeführt werden - bei Normaldruck hätte die Reaktionsmischung einen Siedepunkt, der unterhalb des vorstehend angegebenen Temperaturbereichs liegt. Im nachfolgenden Verfahrensschritt wird das Hydrolysegemisch aus dem Hydrolysereaktor zur destillativen Auftrennung in eine Destillationskolonne gegeben. In letzterer liegt ein niedrigerer Druck als vergleichsweise in dem Hydrolysereaktor vor. Somit wird beim Einführen des Hydrolysegemischs in die Destillationskolonne letzteres schlagartig entspannt (abrupter Druckverlust). Die Folge ist ein heftiges Schäumen des in die Destillationskolonne eingeführten Hydrolysegemischs. Das Schäumen hat stark negative Auswirkungen auf die Trennleistung der Destillationseinbauten der Destillationskolonne, da das Schäumen mit einer intensiven Rückvermischung der bevorzugt im Gegenstrom geführten internen Gas- und Flüssigkeitsströme in der Destillationskolonne verbunden ist. Außerdem wird der fluiddynamische Arbeitsbereich durch das Schäumen stark eingeschränkt, da durch den Schaum ein nicht zu tolerierender Druckverlust in der Kolonne verursacht wird. Zur Verhinderung der Schaumbildung kann in der Destillationskolonne, oberhalb der Zufuhrstelle für das Hydrolysegemisch, ein handelsübliches Antischaummittel hinzugegeben werden. Dadurch wird das Schäumen verhindert bzw. stark eingeschränkt, so daß die vorstehend beschriebenen negativen Konsequenzen des Schäumens ausgeschlossen werden. Als geeignete Antischaummittel können beispielsweise Silikonöle eingesetzt werden. Nachteilig wirken sich die Kosten für die Antischaummittel auf die Wirtschaftlichkeit des Verfahrens aus. Andererseits werden mit der Zugabe der handelsüblichen Antischaummittel Fremdstoffe in das Verfahren eingetragen, die sich in der Regel nachteilig auf die Produktqualität auswirken. Da die Abbauprodukte der Antischaummittel aus dem Prozeß ausgeschleust werden müssen, entstehen erhebliche Entsorgungskosten bei der Reinigung der entsprechenden Abwässer.

Aufgabe der vorliegenden Erfindung ist es, ein Verfahren bereitzustellen, bei dem die Schaumbildung verhindert wird, ohne daß dabei hohe Kosten und eine nachteilige Beeinflussung der Produktqualität zu verzeichnen sind. Außerdem sollen die Entsorgungskosten für das Antischaummittel minimiert werden. Das Verfahren soll einfach und praktisch durchführbar sein.

Die Lösung dieser Aufgabe geht aus von dem Verfahren zur Gewinnung von wasserfreier oder weitgehend wasserfreier Ameisensäure, bei dem man
i) Methylfomiat der Hydrolyse unterwirft,
ii) vom erhaltenen Hydrolysegemisch Methanol sowie überschüssiges Methylformiat abdestilliert,
iii) das Ameisensäure und Wasser enthaltende Sumpfprodukt der Destillation ii) in einer Flüssig-flüssig-Extraktion mit einem hauptsächlich die Ameisensäure aufnehmenden Extraktionsmittel extrahiert und dabei als Extraktionsmittel ein Carbonsäureamid der allgemeinen Formel I 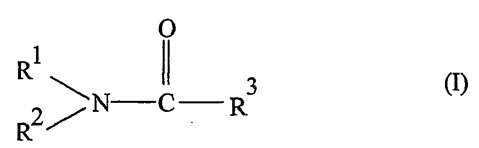 einsetzt, in der die Reste R¹ und R² Alkyl-, Cycloalkyl-, Aryl- oder Aralkylgruppen bedeuten oder R¹ und R² gemeinsam zusammen mit dem N-Atom einen heterocyclischen 5- oder 6-Ring ausbilden und in der nur einer der Reste eine Arylgruppe ist und in der R³ für Wasserstoff oder eine C₁-C₄-Alkylgruppe steht.
iv) die hierbei erhaltene, Ameisensäure, Extraktiosmittel und eine Teilmenge des Wassers aufweisende Extraktphase einer Destillation unterwirft,
v) das bei dieser Destillation erhaltene, Wasser und eine Teilmenge der Ameisensäure enthaltende Kopfprodukt in den unteren Teil der Destillationseinrichtung der Stufe ii) zurückführt,
vi) das vorwiegend Extraktionsmittel und Ameisensäure enthaltende Sumpfprodukt der Destillationsstufe iv) destillativ in wasserfreie oder weitgehend wasserfreie Ameisensäure und das Extraktionsmittel auftrennt und
vii) das die Stufe vi) verlassende Extraktionsmittel in den Verfahrensgang zurückführt.

Das erfindungsgemäße Verfahren ist dann dadurch gekennzeichnet, daß man einen Teilstrom des eingesetzten Extraktionsmittels aus dem Prozeß entnimmt und diesen der für die Durchführung der Stufe ii) vorgesehenen Destillationseinrichtung zuführt, wobei sich die entsprechende Zufuhrstelle für den Teilstrom des Extraktionsmittels in der Destillationseinrichtung oberhalb der Zufuhrstelle des Hydrolysegemischs und unterhalb der Entnahmestelle für Methanol befindet.

Unter weitgehend wasserfreier Ameisensäure soll Ameisensäure verstanden werden, die bis maximal 30 %, bevorzugt bis maximal 15 %, Wasser enthält.

Das in die Destillationseinrichtung zur Durchführung der Stufe ii) eingeführte Extraktionsmittel verhindert das Schäumen. Alle der in Frage kommenden Extraktionsmittel der allgemeinen Formel I sind im Vergleich zu den Komponenten des Hydrolysegemischs (Methylformiat, Wasser, Methanol, Ameisensäure) Schwersieder. Das in die Destillationskolonne zur Durchführung der Stufe ii) eingegebene Extraktionsmittel läuft somit in der Destillationskolonne flüssig ab und gelangt zusammen mit der wässrigen Ameisensäure, die in den Extraktor geführt wird, wieder in den Kreislauf für das Extraktionsmittel.

Die Vorteile des erfindungsgemäßen Verfahrens sind offensichtlich. Es ist nicht mehr notwendig ein teures zusätzliches Antischaummittel einzusetzen, das anschließend wieder aus dem Prozeß entfernt werden muß. Es können somit Kosten für den Erwerb eines Antischaummittels und für die Abwasseraufbereitung, die mit dem Einbringen eines zusätzlichen Antischaummittels verbunden wären, eingespart werden. Grundsätzlich ist es von Vorteil, wenn man in einem Verfahren das Einbringen von Fremdstoffen vermeiden kann. Das Extraktionsmittel als Antischaummittel ist somit eine Komponente, die ohnehin bereits in dem Verfahren vorhanden ist - das Einbringen eines zusätzlichen Antischaummittels wird vermieden.

Als Extraktionsmittel werden bevorzugt N,N-Di-n-butylformamid, N,N-Di-n-butylacetamid, N-Methyl-N-2-heptylformamid, N-n-Butyl-N-2-ethylhexylformamid, N-n-Butyl-N-cyclohexylformamid und/oder N-Ethylformanilid eingesetzt.

In einer bervorzugten Ausführungsform der Erfindung wird der der Destillationseinrichtung zur Durchführung der Stufe ii) zugeführte Teilstrom des eingesetzten Extraktionsmittels dem die Stufe vi) verlassenden Extraktionsmittel entnommen. Prinzipiell kann jedoch das Extraktionsmittel für die Bereitstellung des Teilstroms an jeder beliebigen Stelle des Verfahrens entnommen werden. Als Extraktionsmittel können auch Gemische, die verschiedene erfindungsgemäße Extraktionsmittel enthalten, eingesetzt werden. Das als Antischaummittel verwendete Extraktionsmittel kann neben Extraktionsmittel auch noch andere Komponenten, insbesondere Ameisensäure, Wasser, Methanol und/oder Methylformiat, enthalten. Das Einbringen zusätzlicher, nicht erfindungsgemäßer Antischaummittel ist prinzipiell möglich.

In der Regel werden die Destillationsschritte ii) und iv) in einer einzigen Destillationseinrichtung durchgeführt.

Erfindungsgemäß ist auch die Verwendung eines Carbonsäureamids der allgemeinen Formel I, 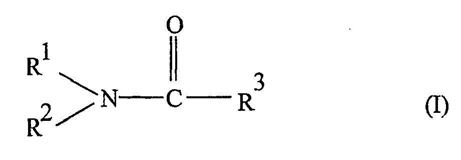 in der die Reste R¹ und R² Alkyl-, Cycloallcyl-, Aryl- oder Aralkylgruppen bedeuten oder R¹ und R² gemeinsam zusammen mit dem N-Atom einen heterocyclischen 5- oder 6-Ring ausbilden und in der nur einer der Reste eine Arylgruppe ist und in der R³ für Wasserstoff steht, in dem vorstehend beschriebenen Verfahren als Antischaummittel bei der destillativen Auftrennung des Methylformiat, Wasser, Ameisensäure und Methanol enthaltenden Hydrolysegemischs sowie als Extraktionsmittel für die Flüssig-flüssig-Extraktion der Ameisensäure.

Erfindungsgemäß wird auch eine Vorrichtung zur Durchführung des vorstehend erläuterten Verfahrens bereitgestellt. Diese enthält
α) einen Synthesereaktor
β) einen Hydrolysereaktor,
χ) eine Destillationseinrichtung zur Durchführung der Stufe ii),
δ) eine Destillationseinrichtung zur Durchführung der Stufe iv)
ε) eine Extraktionseinrichtung,
φ) eine Destillationseinrichtung zur Durchführung der Stufe vi) und
γ) eine Verbindungsleitung für die Zuführung eines Teilstroms des Extraktionsmittels in die für die Durchführung der Stufe ii) vorgesehene Destillationskolonne.

Als Synthesereaktor soll eine Einrichtung verstanden werden, in der einerseits die Synthese von Methylformiat erfolgt (meist in einem entsprechenden Reaktor) und gegebenenfalls andererseits noch eine Trennung des erhaltenen Synthesegemischs (meist in einer dem Reaktor nachgeschalteten Destillationseinrichtung) durchgeführt wird. Auch als Hydrolysereaktor kann jeder beliebige zur Hydrolyse von Methylfomiat geeignete Reaktor eingesetzt werden. Die Destillationseinrichtungen sind in der Regel als Kolonnen ausgebildet. Als Extraktionseinrichtung wird vorzugsweise eine Flüssig-flüssig-Extraktionskolonne eingesetzt. Die Verbindungsleitung für die Zuführung eines Teilstroms des Extraktionsmittels in die für die Durchführung der Stufe ii) vorgesehene Destillationskolonne ist in der Regel als Rohr ausgebildet, welches zwischen der Destillationseinrichtung 2 zur Durchführung der Stufe ii) und einem Ablaufrohr für Extraktionsmittel, das die Destillationseinrichtung zur Durchführung der Stufe iv) verläßt, angeordnet ist.

In einer bevorzugten Ausführungsform der Erfindung ist die Destillationseinrichtung zur Durchführung der Stufe ii) und die Destillationseinrichtung zur Durchführung der Stufe iv) in einer einzigen Destillationseinrichtung angeordnet. Letztere ist in der Regel als Kolonne ausgebildet.

Die anliegende Zeichnung zeigt
- in Figur 1 und Figur 2: Schemata von Anlagen zur Herstellung von wasserfreier oder weitgehend wasserfreier Ameisensäure nach dem Stand der Technik, und
- in Figur 3 und Figur 4: Schemata von Anlagen zur Herstellung von wasserfreier oder weitgehend wasserfreier Ameisensäure gemäß dem erfindungsgemäßen Verfahren.

Die über, unter bzw. neben den Verbindungsleitungen bzw. Pfeilen eingetragenen Bezugszeichen entsprechen den Komponenten, die in der Regel den Hauptanteil des entsprechenden Stroms bilden. Da die Zusammensetzung in den Strömen variieren kann, sollen diese Zahlen lediglich als Richtwert dienen. Dabei bedeuten 21 Methylformiat, 22 Wasser, 23 Ameisensäure, 24 Methanol, 25 Extraktionsmittel und 27 Kohlenmonoxid.

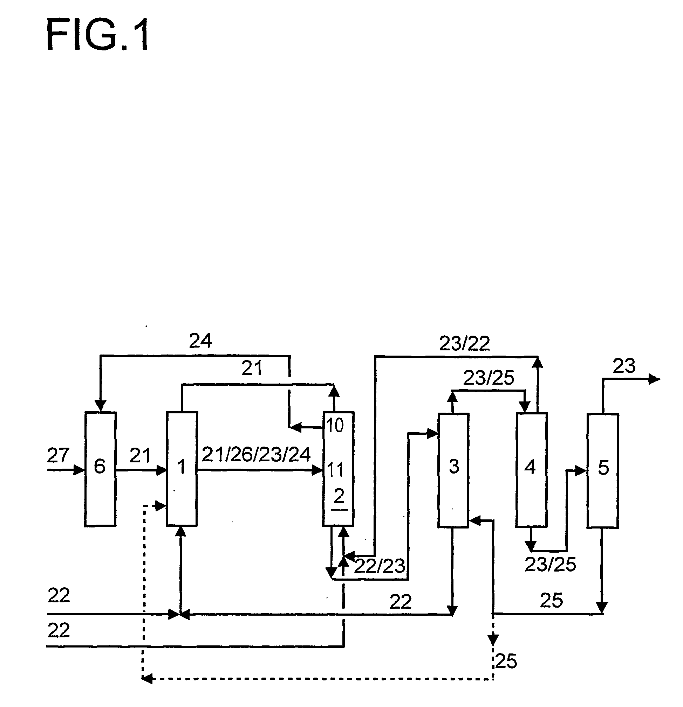

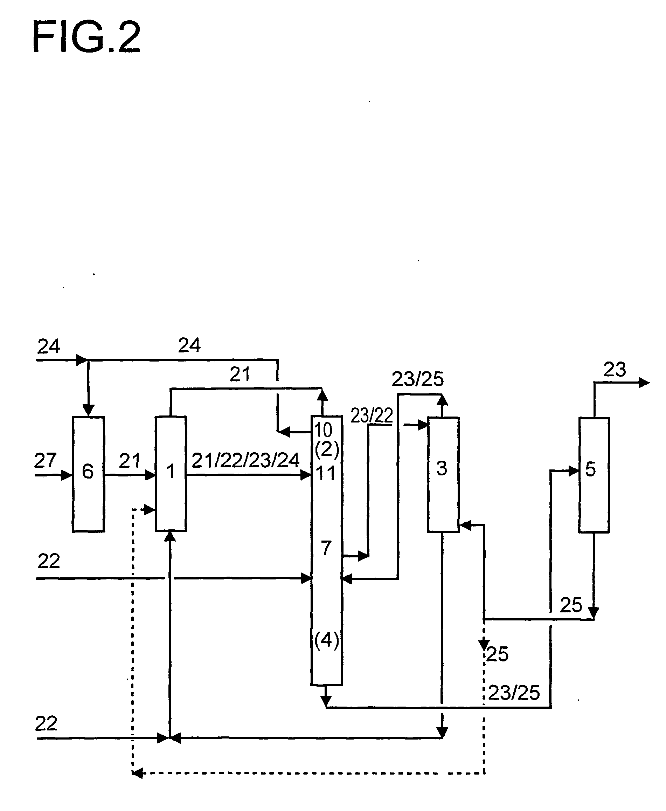

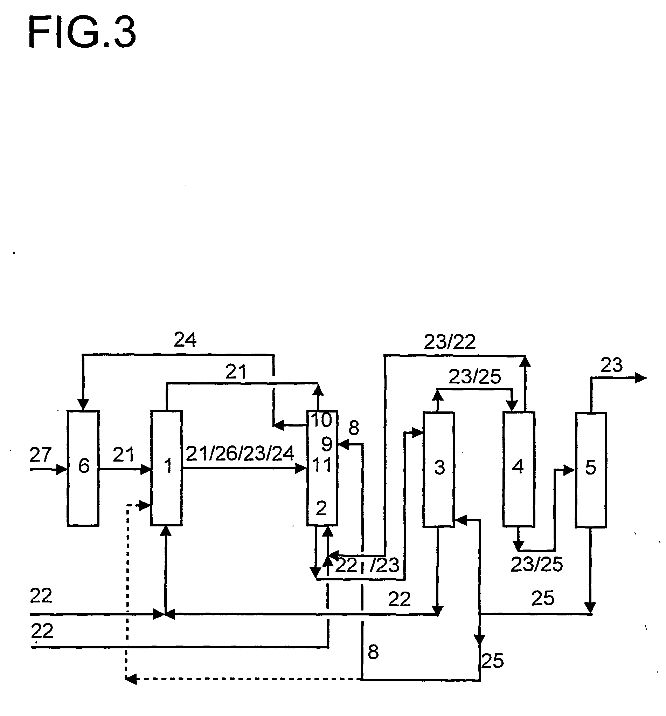

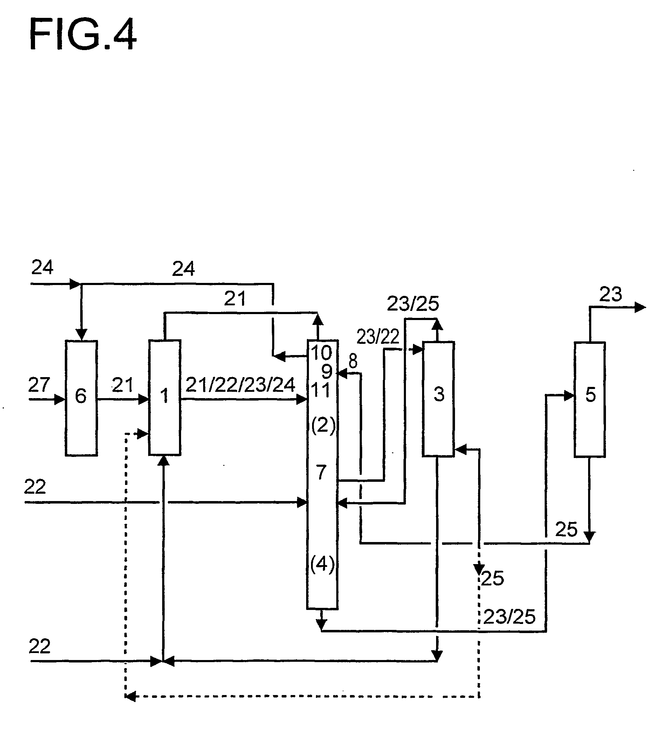

Den in Fig. 1 und Fig. 2 schematisch aufgezeigten Anlagen zur Durchführung des Verfahrens nach dem Stand der Technik und den in Fig. 3 und Fig. 4 schematisch aufgezeigten Anlagen zur Durchführung des erfindungsgemäßen Verfahrens ist gemeinsam, daß diese einen Synthesereaktor 6, einen Hydrolysereaktor 1, eine Destillationseinrichtung 2 zur Durchführung der Stufe ii), eine Destillationseinrichtung 4 zur Durchführung der Stufe iv), eine Extraktionseinrichtung 3 und eine Destillationseinrichtung zur Durchführung der Stufe vi) aufweisen. Die Destillationseinrichtungen 2 und 4 können in einer gemeinsamen Destillationseinrichtung 7 angeordnet sein.

Im Gegensatz zu den in Fig. 1 und Fig. 2 gezeigten Anlagen zum Stand der Technik enthalten die in Fig. 3 und Fig. 4 dargestellten Anlagen zur Durchführung des erfindungsgemäßen Verfahrens eine Verbindungsleitung 8 für die Zuführung eines Teilstroms des Extraktionsmittels in die für die Durchführung der Stufe iv) vorgesehene Destillationskolonne 2. Dieser wird dem aus der Destillationseinrichtung 5 ablaufenden Strom des Extraktionsmittels entnommen und vorteilhafterweise gekühlt. Die Verbindungsleitung 8 wird an der Zufuhrstelle 9 in die Destillationseinrichtung 2 zur Durchführung der Stufe ii) eingeführt. Diese Zufuhrstelle 9 für den Teilstrom des Extraktionsmittels ist in der Destillationseinrichtung oberhalb der Zufuhrstelle 11 für das Hydrolysegemisch und unterhalb der Entnahmestelle 10 für Methanol angeordnet. In der Regel befindet sich die Zufuhrstelle 9 für das Extraktionsmittel 2 bis 40 theoretische Trennstufen, insbesondere 5 bis 20 theoretische Trennstufen, oberhalb der Zufuhrstelle 11 für das Hydrolysegemisch.

Die Erfindung soll im folgenden anhand eines Ausführungsbeispiels näher erläutert werden.

### Beispiel

Der erfindungsgemäße Beispielversuch wird in einer Anlage durchgeführt, die schematisch in Fig. 4 aufgezeigt ist. Es werden kontinuierlich 5,3 kg/h wäßrige Ameisensäure hergestellt. Die verwendete Destillationseinrichtung 7, die als Technikumskolonne ausgebildet ist, hat einen Durchmesser von 100 mm und ist mit 100 Glockenböden ausgestattet. Der entsprechende Reaktoraustrag beträgt 20 kg/h. Die Aufgabe des Hydrolysegemischs erfolgt auf dem fünfundvierzigsten Boden, das Antischaummittel wird auf dem fünfundsechsigsten Boden zugegeben. Die Kolonne wird unter Atmosphärendruck betrieben. Als Extraktionsmittel wird N,N-Di-n-butylformamid eingesetzt. Es wird 20 Böden oberhalb des Zulaufs 11 für das Hydrolysegemisch ein Extraktionsmittelstrom von 200 g/h zugeführt. Dadurch kann die Schaumbildung vollständig unterdrückt. Durch diese Maßnahme erreichen die Böden oberhalb der Zufuhrstelle 11 für das Hydrolysegemisch den bei Zusatz von Antischaummitteln üblichen Wirkungsgrad von 0,7 theoretischen Trennstufen pro praktischem Boden. Die Druckverlust beträgt 2 mbar pro Boden.

### zum Vergleich:

Ohne die Zugabe eines Antischaummittels tritt bei der Destillationseinrichtung 2, oberhalb der Zufuhrstelle des Hydrolysegemischs 11 ein heftiges Schäumen auf Durch das Schäumen sinkt der Bodenwirkungsgrad der mit Schaum gefüllten Böden auf 0,2 theoretische Trennstufen pro praktischem Boden. Der Druckverlust beträgt 3,5 mbar pro Boden.

Der vorstehende Versuch zeigt, daß sich das eingesetzte Extraktionsmittel als Antischaummittel bei dem erfindungsgemäßen Verfahren eignet.

## Patentansprüche

1. Verfahren zur Gewinnung von wasserfreier oder weitgehend wasserfreier Ameisensäure, bei dem man,
i) Methylformiat der Hydrolyse unterwirft,
ii) vom erhaltenen Hydrolysegemisch Methanol sowie überschüssiges Methylformiat abdestilliert,
iii) das Ameisensäure und Wasser enthaltende Sumpfprodukt der Destillation (ii) in einer Flüssig-flüssig-Extraktion mit einem hauptsächlich die Ameisensäure aufnehmenden Extraktionsmittel extrahiert und dabei als Extraktionsmittel ein Carbonsäureamid der allgemeinen Formel I 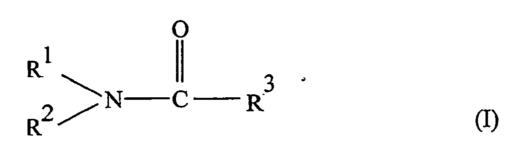 einsetzt, in der die Reste R¹ und R² Alkyl-, Cycloalkyl-, Aryl- oder Aralkylgruppen bedeuten oder R¹ und R² gemeinsam zusammen mit dem N-Atom einen heterocyclischen 5- oder 6-Ring ausbilden und in der nur einer der Reste eine Arylgruppe ist und in der R³ für Wasserstoff oder eine C₁-C₄-Alkylgruppe steht,
iv) die hierbei erhaltene Ameisensäure, Extraktionsmittel und eine Teilmenge des Wassers aufweisende Extraktphase einer Destillation unterwirft,
v) das bei dieser Destillation erhaltene Wasser und eine Teilmenge der Ameisensäure enthaltende Kopfprodukt in den unteren Teil der Destillationseinrichtung der Stufe ii) zurückführt,
vi) das vorwiegend Extraktionsmittel und Ameisenäure enthaltende Sumpfprodukt der Destillationsstufe iv) destillativ in wasserfreie oder weitgehend wasserfreie Ameisensäure und das Extraktionsmittel auftrennt und
vii) das die Stufe vi) verlassende Extraktionsmittel in den Verfahrensgang zurückführt,
**dadurch gekennzeichnet, daß** man einen Teilstrom des eingesetzten Extraktionsmittels aus dem Prozeß entnimmt und diesen der für die Durchführung der Stufe ii) vorgesehenen Destillationseinrichtung zuführt, wobei sich die entsprechende Zufuhrstelle für den Teilstrom des Extraktionsmittels in der Destillationseinrichtung oberhalb der Zufuhrstelle des Hydrolysegemischs und unterhalb der Entnahmestelle für Methanol befindet.

2. Verfahren nach Anspruch 1, **dadurch gekennzeichnet, daß** als Extraktionsmittel N,N-Di-n-butylformamid, N,N-Di-n-butylacetamid, N-Methyl-N-2-heptylformamid, N-n-Butyl-N-2-ethylhexylformamid, N-n-Butyl-N-cyclohexylformamid und/oder N-Ethylformanilid eingesetzt wird.

3. Verfahren nach Anspruch 1 oder 2, **dadurch gekennzeichnet, daß** der Teilstrom des eingesetzten Extraktionsmittels dem die Stufe vi) verlassenden Extraktionsmittel entnommen wird.

4. Verfahren nach einem der Ansprüche 1 bis 3, **dadurch gekennzeichnet, daß** die Destillationsschritte ii) und iv) in einer einzigen Destillationseinrichtung durchgeführt werden.

5. Verwendung eines Carbonsäureamids der allgemeinen Formel I 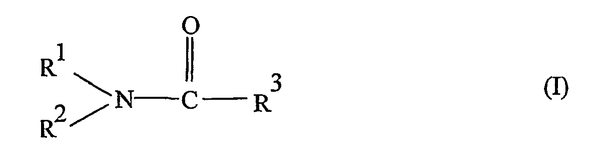 in der die Reste R¹ und R² Alkyl-, Cycloalkyl-, Aryl- oder Aralkylgruppen bedeuten oder R¹ und R² gemeinsam zusammen mit dem N-Atom einen heterocyclischen 5-oder 6-Ring ausbilden und in der nur einer der Reste eine Arylgruppe ist und in der R³ für Wasserstoff oder eine C₁-C₄-Alkylgruppe steht,
in dem Verfahren gemäß einem der Ansprüche 1 bis 4 als Antischaummittel bei der destillativen Auftrennung des Methylformiat, Wasser, Ameisensäure und Methanol enthaltenden Hydrolysegemischs sowie als Extraktionsmittel für die Flüssig-flüssig-Extraktion der Ameisensäure.

6. Vorrichtung zur Durchführung des Verfahrens gemäß eines der Ansprüche 1 bis 4, enthaltend
α) einen Synthesereaktor (6),
β) einen Hydrolysereaktor (1),
χ) eine Destillationseinrichtung (2) zur Durchführung der Stufe ii),
δ) eine Destillationseinrichtung (4) zur Durchführung der Stufe iv),
ε) eine Extraktionseinrichtung (3),
φ) eine Destillationseinrichtung (5) zur Durchführung der Stufe vi) und
γ) eine Verbindungsleitung (8) für die Zuführung eines Teilstroms des Extraktionsmittels in die für die Durchführung der Stufe ii) vorgesehene Destillationskolonne (2).

7. Vorrichtung nach Anspruch 6, **dadurch gekennzeichnet, daß** die Destillationseinrichtung (2) zur Durchführung der Stufe ii) und die Destillationseinrichtung (4) zur Durchführung der Stufe iv) in einer einzigen Destillationseinrichtung angeordnet sind.

## Claims

1. A process for obtaining anhydrous or substantially anhydrous formic acid, in which
i) methyl formate is subjected to hydrolysis,
ii) methanol and excess methyl formate are distilled off from the resultant hydrolysis mixture,
iii) the bottom product from distillation (ii), comprising formic acid and water, is extracted in a liquid-liquid extraction with an extractant which principally takes up the formic acid, and the extractant employed here is a carboxamide of the general formula I 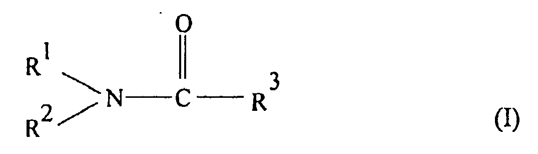 where the radicals R¹ and R² are alkyl, cycloalkyl, aryl or aralkyl groups, or R¹ and R² jointly, together with the N atom, form a heterocyclic 5- or 6-membered ring, and where only one of the radicals is an aryl group, and where R³ is hydrogen or a C₁-C₄-alkyl group,
iv) the resultant extract phase, comprising formic acid, extractant and some of the water, is subjected to distillation,
v) the top product obtained in this distillation, which comprises water and some of the formic acid, is fed back into the lower part of the distillation device in step ii),
vi) the bottom product from distillation step iv), which comprises predominantly extractant and formic acid, is separated by distillation into anhydrous or substantially anhydrous formic acid and the extractant, and
vii) the extractant leaving step vi) is fed back into the process,
which comprises removing a sub-stream of the extractant employed from the process and feeding it to the distillation device provided for carrying out step ii), with the corresponding feed point for the sub-stream of the extractant in the distillation device being above the feed point of the hydrolysis mixture and below the removal point of methanol.

2. The process according to claim 1, wherein the extractant employed is N,N-di-n-butylformamide, N,N-di-n-butylacetamide, N-methyl-N-2-heptylformamide, N-n-butyl-N-2-ethylhexylformamide, N-n-butyl-N-cyclohexylformamide and/or N-ethylformanilide.

3. The process according to claim 1 or 2, wherein the sub-stream of the extractant employed is taken from the extractant leaving step vi).

4. The process according to any one of claims 1 to 3, wherein distillation steps ii) and iv) are carried out in a single distillation device.

5. The use of a carboxamide of the general formula I 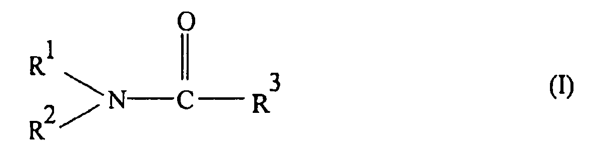 where the radicals R¹ and R² are alkyl, cycloalkyl, aryl or aralkyl groups, or R¹ and R² jointly, together with the N atom, form a heterocyclic 5- or 6-membered ring, and where only one of the radicals is an aryl group, and where R³ is hydrogen or a C₁-C₄-alkyl group,
in the above-described process according to any one of claims 1 to 4 as antifoam in the distillative separation of the hydrolysis mixture comprising methyl formate, water, formic acid and methanol, and as extractant for the liquid-liquid extraction of the formic acid.

6. An apparatus for carrying out the process according to any one of claims 1 to 4, comprising
α) a synthesis reactor (6),
β) a hydrolysis reactor (1),
χ) a distillation device (2) for carrying out step ii),
δ) a distillation device (4) for carrying out step iv),
ε) an extraction device (3),
φ) a distillation device (5) for carrying out step vi), and
γ) a connecting line (8) for feeding a sub-stream of the extractant into the distillation column (2) provided for carrying out step ii).

7. The apparatus according to claim 6, wherein the distillation device (2) for carrying out step ii) and the distillation device (4) for carrying out step iv) are arranged in a single distillation device.

## Revendications

1. Procédé d'obtention d'acide formique anhydre ou largement anhydre, dans lequel :
i) on soumet le méthylformiate à une hydrolyse,
ii) on sépare le méthanol du mélange d'hydrolyse obtenu, ainsi que le méthylformiate en excès, par distillation,
iii) on extrait le produit de fond de colonne de la distillation (ii) contenant l'acide formique et l'eau lors d'une extraction liquide-liquide avec un agent d'extraction absorbant principalement l'acide formique et on utilise pour cela comme agent d'extraction un amide d'acide carboxylique de formule générale I : 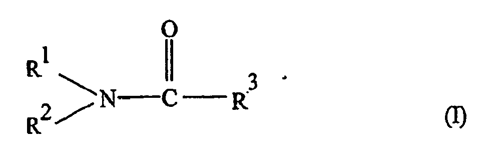 dans laquelle les radicaux R¹ et R² signifient des groupes alkyle, cycloalkyle, aryle ou aralkyle ou R¹ et R² forment ensemble avec l'atome de N un noyau hétérocyclique à 5 ou 6 chaînons et dans laquelle seul un des radicaux est un groupe aryle et dans laquelle R³ représente l'hydrogène ou un groupe alkyle en C₁-C₄,
iv) on soumet la phase d'extraction obtenue ici présentant l'acide formique, l'agent d'extraction et une quantité partielle de l'eau à une distillation,
v) on recycle le produit de tête contenant l'eau obtenue lors de la distillation et une quantité partielle de l'acide formique dans la partie inférieure de l'unité de distillation de l'étape ii),
vi) on sépare le produit de fond de colonne contenant principalement l'agent d'extraction et l'acide formique de l'étape de distillation iv) par distillation dans l'acide formique anhydre ou largement anhydre ainsi que l'agent d'extraction, et
vii)on recycle l'agent d'extraction quittant l'étape vi) dans le processus du procédé,
**caractérisé en ce qu'**on prélève une partie du courant de l'agent d'extraction utilisé hors du processus et on conduit celle-ci à l'unité de distillation prévue pour l'exécution de l'étape ii), le point d'amenée correspondant pour la partie du courant de l'agent d'extraction dans l'unité de distillation se trouvant au-dessus du point d'amenée du mélange d'hydrolyse et en dessous du point de prélèvement du méthanol.

2. Procédé selon la revendication 1, **caractérisé en ce qu'**on utilise comme agent d'extraction le N,N-di-n-butylformamide, le N,N-di-n-butylacétamide, le N-méthyl-N-2-heptylformamide, le N-n-butyl-N-2-éthylhexylformamide, le N-n-butyl-N-cyclohexylformamide et/ou le N-éthylformanilide.

3. Procédé selon la revendication 1 ou 2, **caractérisé en ce que** la partie de courant de l'agent d'extraction utilisé est prélevée de l'agent d'extraction quittant l'étape vi).

4. Procédé selon l'une des revendications 1 à 3, **caractérisé en ce que** les étapes de distillation ii) et iv) sont réalisées dans une seule et unique unité de distillation.

5. Utilisation d'un amide d'acide carboxylique de formule générale I : 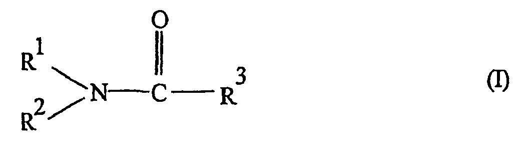 dans laquelle les radicaux R¹ et R² signifient des groupes alkyle, cycloalkyle, aryle ou aralkyle ou R¹ et R² forment ensemble avec l'atome de N un noyau hétérocyclique à 5 ou 6 chaînons et dans laquelle seul un des radicaux est un groupe aryle et dans laquelle R³ représente l'hydrogène ou un groupe alkyle en C₁-C₄,
dans le procédé selon l'une des revendications 1 à 4 comme agent anti-mousse lors de la séparation par distillation du mélange d'hydrolyse contenant le méthylformiate, l'eau, l'acide formique et le méthanol, ainsi que comme agent d'extraction pour l'extraction liquide-liquide de l'acide formique.

6. Dispositif pour l'exécution du procédé selon l'une des revendications 1 à 4, contenant :
α) un réacteur de synthèse (6),
β) un réacteur d'hydrolyse (1),
χ) une unité de distillation (2) pour l'exécution de l'étape ii),
δ) une unité de distillation (4) pour l'exécution de l'étape iv),
ε) une unité d'extraction (3),
φ) une unité de distillation (5) pour l'exécution de l'étape vi), et
γ) une conduite de connexion (8) pour l'amenée d'une partie du courant de l'agent d'extraction dans la colonne de distillation (2) prévue pour l'exécution de l'étape ii).

7. Dispositif selon la revendication 6, **caractérisé en ce que** l'unité de distillation (2) est disposée pour l'exécution de l'étape ii), et l'unité de distillation (4) l'est pour l'exécution de l'étape iv) dans une seule et unique unité de distillation.
